# FASCICULE DE BREVET EUROPEEN

(11) **EP 0 892 642 B1**
(45) Date de publication et mention de la délivrance du brevet: **14.08.2002**
(21) Numéro de dépôt: 97933720.1
(22) Date de dépôt: 11.07.1997
(51) Int. Cl.: A61K 35/78, A61K 7/06, A61K 7/48, A61P 29/00

(54) **UTILISATION DANS UNE COMPOSITION D'UN EXTRAIT D'AU MOINS UNE LABIEE DU GENRE ROSMARINARUS, CULTIVEE IN VITRO**
VERWENDUNG EINES EXTRAKTES MINDESTENS EINER IN VITRO KULTIVIERTEN LABIATE DER GATTUNG ROSMARINARUS IN EINER ZUSAMMENSETZUNG
USE IN A COMPOSITION OF AN EXTRACT OF AT LEAST ONE LABIATE OF THE GENUS ROSMARINARUS, CULTURED IN VITRO

(30) Priorité: 30.07.1996 FR 9609593
(43) Date de publication de la demande: 27.01.1999
(73) Titulaire: L'OREAL, 75008 Paris (FR)
(72) Inventeur: BRETON, Lionel, F-78000 Versailles (FR); MARTIN, Richard, F-37210 Rochecorbon (FR); PINEAU, Nathalie, F-86000 Poitiers (FR)
(74) Mandataire: Tezier Herman, Béatrice
(86) Numéro de dépôt international: FR9701288
(87) Numéro de publication internationale: WO9804276

(56) Documents cités:
- WO-A-93/25209
- DE-A- 3 536 342
- FR-A- 2 504 551
- FR-A- 2 662 078
- PATENT ABSTRACTS OF JAPAN vol. 013, no. 290 (C-614), 5 juillet 1989 & JP 01 085087 A (NARISU KESHOHIN:KK), 30 mars 1989,
- PATENT ABSTRACTS OF JAPAN vol. 013, no. 315 (C-619), 18 juillet 1989 & JP 01 102027 A (NITTO DENKO CORP), 19 avril 1989,
- MAHDU JAIN ET AL.: "IN VITRO PRODUCTION OF ESSENTIAL OIL FROM PROLIFERATING SHOOTS OF ROSMARINUS OFFICINALIS." PLANTA MEDICA, vol. 57, no. 2, avril 1991, pages 122-124, XP000673353
- DEREK V. BANTHROPE ET AL.: "STIMULATION OF ACCUMULATION OF TERPENOIDS BY CELL SUSPENSIONS OF LAVANDULA ANGUSTIFOLIA FOLLOWING PRE-TREATMENT OF PARENT CALLUS." PHYTOCHEMISTRY, vol. 40, no. 1, 1995, pages 83-87, XP000671795
- ELIZABETH A. OFFORD ET AL.: "ROSEMARY COMPONENTS INHIBIT BENZO[A]PYRENE-INDUCED GENOTOXICITY IN HUMAN BRONCHIAL CELLS." CARCINOGENESIS, vol. 16, no. 9, 1995, pages 2057-2062, XP000673354

## Description

La présente invention a pour objet, dans son aspect le plus général un extrait d'au moins un végétal de la famille des Labiées du genre Rosmarinus ledit végétal étant cultivé *in vitro.*

Elle a également pour objet une composition cosmétique ou pharmaceutique comprenant à titre de principe actif au moins une quantité efficace d'un tel extrait, l'utilisation dans une composition cosmétique ou pour la préparation d'une composition pharmaceutique, à titre de principe actif pour combattre les désordres faisant intervenir un processus inflammatoire ou un processus allergique, d'une quantité efficace d'au moins un tel extrait et un procédé de traitement cosmétique faisant intervenir un tel extrait.

Par la suite dans le texte l'expression "extrait de Labiées du genre Rosmarinus " aura la même signification que "extrait d'au moins un végétal de la famille des Labiées du genre Rosmarinus ".

L'inflammation (ou processus inflammatoire) est un ensemble de réactions biologiques qu'on retrouve dans toute l'échelle animale. Chez l'homme, deux malades sur trois présentent un syndrome inflammatoire. L'inflammation peut être localisée. Elle peut se définir comme la première réponse à toute agression locale par une série de réactions non spécifiques déclenchées quelle que soit la cause initiale et se déroulant en trois temps : vasculaire, cellulo-vasculaire et fibrose tissulaire.
Gonflement, douleur, rougeur, chaleur sont les termes que l'on peut utiliser pour décrire l'inflammation localisée. Ceux-ci sont généralement dus à l'infiltration des tissus blessés par un oedème et/ou à la vasodilatation des capillaires.

Les signes de l'inflammation peuvent aller jusqu'à la fièvre, un état de malaise général et/ou une augmentation de la concentration de certaines protéines de plasma sanguin.
C'est un phénomène qui implique entre autres, une série de réactions cellulaires locales et la libération de cytokines et autres médiateurs tels que la substance P, les prostaglandines, les leukotriènes, la bradykinine, l'histamine ou encore la sérotonine.
Elle se manifeste par une modification du flux sanguin avec, au niveau du site agressé, une augmentation de la perméabilité vasculaire entraînant une fuite de protéines plasmatiques et de cellules vers le fluide extracellulaire, ainsi qu'une extravasation de leucocytes, principalement des leucocytes neutrophiles et de macrophages vers le site inflammatoire.

Ces phénomènes sont en fait le résultat de l'action des médiateurs de l'inflammation.
Parmi les facteurs impliqués dans ces phénomènes inflammatoires, on peut citer les cytokines dont en particulier l'interleukine 1-α, l'interleukine 1-β, l'interleukine 6, les facteurs de nécrose tumorale α et β (TNF-α et -β), les chémokines comme l'interleukine 8 ou le facteur chimiotactique et activateur des monocytes (MCAF),
ou encore d'autres facteurs chimiotactiques responsables du recrutement des cellules lymphocytaires, monocytaires, de Langerhans ou basophiles au niveau du site inflammatoire, tels que les leukotriènes B-4, ou encore d'autres facteurs impliqués dans la cascade inflammatoire, tels que l'acide arachidonique, ou les prostaglandines, dont en particulier les prostaglandines E2.

Les phénomènes inflammatoires sont associés à de nombreuses pathologies.
On peut citer à titre d'exemple les affections rhumatismales telles que le rhumatisme articulaire aigu, la polyarthrite rhumatoïde, les affections pulmonaires telles que l'emphysème, les affections articulaires telles que l'arthrose, les tendinites, la périarthrite, les spondylarthropathies ou les atteintes articulaires des entérophaties chroniques, les allergies, les phases inflammatoires de l'alopécie, les affections cutanées telles que les peaux sensibles, les érythèmes, en particulier dus aux ultraviolets, le prurit, l'érythème noueux, l'urticaire, la mastocytose systémique, le psoriasis, les piqûres d'insectes, d'autres affections dermatologiques comme la polychondrite atrophiante, l'érythèmalgie, la nécrobiose lipoïdique ou encore le lupus érithèmateux disséminé.

Dans les processus allergiques, les facteurs précoces impliqués sont également des cytokines notamment les interleukines 1α et 1β et le facteur de nécrose tumorale de type α (TNF α).
A la différence d'une réaction inflammatoire, une réaction allergique est un phénomène qui répond à un facteur extérieur ou allergène. Il s'agit alors d'un processus spécifiquement immunologique qui ne se produit que lorsqu'un allergène est présent et qui ne touche que des sujets sensibilisés.
Mais, la résultante finale d'une réaction allergique se traduit aussi par une réaction inflammatoire aiguë, associée généralement à un oedème.

Quel que soit le phénomène envisagé, il existe un point commun à tous ces mécanismes qui se traduit par une réaction inflammatoire dont la facette terminale se mesure par la libération par les cellules mastocytaires de la peau d'au moins un médiateur de l'inflammation tel que l'histamine, la sérotonine, l'héparine, les leukotriènes, les prostaglandines, les cytokines, le monoxyde d'azote ou des espèces oxygénées réactives.
On recherche depuis de nombreuses années, dans l'industrie pharmaceutique, des substances permettant de traiter l'inflammation ou l'allergie. A cet égard, nombreuses sont celles qui ont déjà été décrites, connue dans la littérature sous les appellations d'anti-inflammatoires stéroïdiens ou non-stéroïdiens (AIS ou AINS) et dont on trouvera une description dans, par exemple, l'ouvrage de Schorderet et Dayer "Pharmacologie, Des concepts fondamentaux aux applications thérapeutiques", 1992, chapitre 37, pages 541-561, 2^{ième} édition, Frison-Roche/Slatkine éditeurs.

Outre que les anti-inflammatoires connus présentent souvent des effets secondaires non négligeables, il demeure intéressant de disposer de nouveaux produits à activité anti-inflammatoire.

Le but de la présente invention est donc de pouvoir disposer d'un produit nouveau présentant une activité anti-inflammatoire et pouvant ne pas présenter d'effets secondaires notables.

Ce but et d'autres sont atteints par la présente invention qui a pour objet un extrait d'au moins un végétal de la famille des Labiées du genre Rosmarinus, caractérisé en ce que ledit végétal est cultivé *in vitro.*

L'effet anti-inflammatoire d'un extrait de végétaux de la famille des Labiées est connu en soi. On peut citer à cet égard les demandes de brevet FR-A-2504551, JP-A-7017846, WO-A-9325209, SU-A-1733000, FR-A-2662078, DE-A-3536342, US 5393526. Mahdu Jain et al. (Planta Medica vol. 57, n°2, Avril 1991, pages 122-124) décrit l'utilisation d'huiles essentielles extraites d'organes de Rosmarinus Officinalis Cultivés in vitro en cosmétique et en médecine.

Cependant, de manière surprenante et inattendue la demanderesse a découvert qu'un extrait d'un végétal de la famille des Labiées du genre Rosmarinus, ledit végétal étant cultivé *in vitro,* présente une activité anti-inflammatoire supérieure à celle que présente un extrait de plante entière cultivées *in vivo.* On trouvera par ailleurs dans le texte les résultats d'expériences de fixation aux récepteurs de médiateurs de l'inflammation qui établissent ce fait.

C'est ainsi que l'invention a pour objet un extrait d'au moins un végétal de la famille des Labiées du genre Rosmarinus, caractérisé en ce que le végétal est cultivé *in vitro.*

La pression de sélection imposée par les conditions physico-chimiques lors de la croissance des cellules végétales *in vitro* permet d'obtenir un matériel végétal standardisé et disponible tout au long de l'année contrairement aux plantes cultivées *in vivo.*
Par culture *in vitro*, on entend l'ensemble des techniques connues de l'homme du métier qui permet de manière artificielle l'obtention d'un végétal ou d'une partie d'un végétal.

Le matériel végétal utilisé dans l'invention peut être tout matériel végétal obtenu par culture *in vitro*. On entend ainsi la plante entière ou un organe particulier.
En particulier le matériel végétal peut être des cellules végétales et encore plus particulièrement des cellules végétales indifférenciées (ou dédifférenciées).

Par cellules végétales indifférenciées, on entend toute cellule végétale ne présentant aucun des caractères d'une spécialisation particulière et capable de vivre par elle-même et non en dépendance avec d'autres cellules. Ces cellules végétales indifférenciées sont éventuellement aptes, sous l'effet d'une induction, à toute différenciation conforme à leur génome.
Selon la méthode de culture choisie, et en particulier selon le milieu de culture choisi, il est possible d'obtenir, à partir d'un même explant, des cellules végétales indifférenciées présentant des caractères différents.

La famille des Labiées compte environ 2700 espèces.

L'extrait de l'invention est préparé à partir de matériel végétal d'une Labiée provenant du genre Rosmarinus.

Plus particulièrement, selon l'invention, l'extrait est préparé à partir de matériel végétal provenant de *Rosmarinus officinalis.*

Selon l'invention, l'extrait d'au moins une Labiée du genre Rosmarinus peut être tout extrait préparé à partir de matériel végétal issu de la famille des Labiées du genre Rosmarinus, cultivé *in vitro.*

Toute méthode d'extraction connue de l'homme du métier peut être utilisée pour préparer l'extrait selon l'invention.
On peut, en particulier, citer les extraits alcooliques, notamment éthanoliques ou encore hydroalcooliques.

On peut également utiliser un extrait préparé par la méthode décrite dans la demande de brevet français n° 95-02379 déposée par la demanderesse.
Ainsi, dans une première étape on broie le matériel végétal dans une solution aqueuse à froid, dans une deuxième étape les particules en suspension sont éliminées de la solution aqueuse issue de la première étape, et dans une troisième étape on stérilise la solution aqueuse issue de la deuxième étape. Cette solution aqueuse correspond à l'extrait.
Cet extrait peut alors être lyophilisé.

La première étape peut être avantageusement remplacée par une opération de congélation simple des tissus végétaux (par exemple à -20°C), suivie d'une extraction aqueuse reprenant les deuxième et troisième étapes ci-dessus décrites.

Si le matériel végétal est de la plante entière, on ramène la matière fraîche à traiter en fonction du poids sec pour se mettre dans les mêmes conditions d'extraction que pour l'*in vitro.* Les différentes parties de la plante sont prélevées en fonction du poids relatif de chaque partie de celle-ci.

Le traitement à froid permet de geler les activités enzymatiques, la filtration stérilisante évite la dégradation des actifs par les micro-organismes de l'environnement. Enfin, le véhicule eau est compatible avec les récepteurs *ex vivo* et facilite les formulations cosmétiques ou pharmaceutiques.

Il est connu que les extraits végétaux contiennent des oxydases responsables, entre autres, de l'oxydation desdits extraits. Or une telle oxydation conduit à une coloration marron foncée des extraits et à une odeur âcre rendant ceux-ci peu compatibles avec leur utilisation en cosmétique. Dans cet ordre d'idée on connaît en particulier une laccase dont le poids moléculaire est supérieur à 100000 daltons.

Ainsi, avantageusement, l'extrait obtenu peut être fractionné par toute méthode de fractionnement connue permettant d'éliminer les oxydases et en particulier la polyphénoloxydase. On peut par exemple filtrer l'extrait de l'invention sur une membrane de dialyse afin d'en éliminer les molécules d'un poids moléculaire supérieur à 100000 daltons. Il est également possible de faire subir à l'extrait un fractionnement par précipitations sélectives.

D'autres méthodes permettent de se prémunir des phénomènes d'oxydation. En particulier, l'extrait peut également être stabilisé. Toute méthode de stabilisation connue peut être utilisée selon l'invention. On peut par exemple stabiliser l'extrait de l'invention en y ajoutant de la cystéine à une concentration finale comprise entre 0,5 g/l et 10 g/l et de préférence entre 1,5 g/l et 2,5 g/l.

Bien évidement l'extrait selon l'invention peut être fractionné et stabilisé.

Un exemple de préparation d'extrait utilisable selon l'invention est donné par ailleurs dans les exemples.

L'invention a également pour objet une composition cosmétique ou pharmaceutique, comprenant dans un milieu cosmétiquement ou pharmaceutiquement acceptable, à titre de principe actif, au moins un extrait d'au moins une Labiée du genre Rosmarinus, tel que défini précédement.

La composition pharmaceutique est préférentiellement une composition dermatologique.

La quantité d'extrait contenue dans la composition de l'invention est bien entendu fonction de l'effet recherché et peut donc varier dans une large mesure.

Pour donner un ordre de grandeur, si la composition est une composition cosmétique, elle peut contenir un extrait tel que défini précédement en une quantité représentant de 0,001% à 50% du poids total de la composition et préférentiellement en une quantité représentant de 0,005% à 25% du poids total de la composition.

Pour donner un ordre de grandeur, si la composition est une composition pharmaceutique, elle peut contenir un extrait tel que défini précédement en une quantité représentant de 0,01% à 70% du poids total de la composition et préférentiellement en une quantité représentant de 0,05% à 40% du poids total de la composition.

On a vu préalablement dans le texte des exemples de désordres faisant intervenir un processus inflammatoire.

Ces désordres inflammatoires peuvent être cutanés.

Ainsi, les compositions selon l'invention sont destinées à combattre les désordres cutanés faisant intervenir un processus inflammatoire.

Particulièrement les compositions selon l'invention sont destinées à combattre les affections rhumatismales telles que le rhumatisme articulaire aigu, la polyarthrite rhumatoïde, les affections pulmonaires telles que l'emphysème, les affections articulaires telles que l'arthrose, la tendinite, la périarthrite, les spondylarthropathies ou les atteintes articulaires des entérophaties chroniques, , l'alopécie dans ces phases inflammatoires, les affections cutanées telles que les peaux sensibles, les érythèmes, en particulier dus aux ultraviolets, le prurit, l'érythème noueux, l'urticaire, la mastocytose systémique, le psoriasis, les piqûres d'insectes, ou encore d'autres affections dermatologiques comme la polychondrite atrophiante, l'érythèmalgie, la nécrobiose lipoïdique. On peut encore citer le lupus érithèmateux disséminé.

Encore plus particulièrement les compositions selon l'invention sont destinées à combattre les irritations cutanées et/ou les dartres et/ou les sensations dysesthésiques et/ou les sensations d'échauffement et/ou les prurits de la peau et/ou des muqueuses.

La composition selon l'invention peut être ingérée, injectée ou appliquée sur la peau (sur toute zone cutanée du corps), les cheveux, les ongles ou les muqueuses (buccale, jugale, gingivale, génitale, conjonctive). Selon le mode d'administration, la composition selon l'invention peut se présenter sous toutes les formes galéniques normalement utilisées.

Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.
Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.

Pour l'injection, la composition peut se présenter sous forme de lotion aqueuse, huileuse ou sous forme de sérum. Pour les yeux, elle peut se présenter sous forme de gouttes et pour l'ingestion, elle peut se présenter sous forme de capsules, de granulés, de sirops ou de comprimés.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosasée, le psoriasis, les lichens, les prurits sévères.

La composition selon l'invention peut également consister en des préparations solides constituant des savons ou des pains de nettoyage.

La composition peut aussi être conditionnée sous forme d'une composition pour aérosol comprenant également un agent propulseur sous pression.

La composition selon l'invention peut aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.

La composition peut aussi être à usage bucco-dentaire, par exemple une pâte dentifrice. Dans ce cas, la composition peut contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.
Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose R 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention la composition peut associer au moins un extrait d'au moins une Labiée du genre Rosmarinus à d'autres agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides alpha- et bêta-hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle ;
- les extraits d'autres végétaux et/ou de microorganismes.

Ainsi, selon un mode particulier, l'invention concerne une composition contenant au moins un extrait d'au moins une Labiée du genre Rosmarinus et au moins un agent choisi parmi les agents antibactériens, antiparasitaires, antifongiques, antiviraux, anti-inflammatoires, antiprurigineux, anesthésiques, kératolytiques, anti-radicaux libres, anti-séborrhéiques, antipelliculaires, antiacnéiques, les agents modulant la différenciation et/ou la prolifération et/ou la pigmentation cutanée, les extraits d'autres végétaux et/ou de microorganismes.

L'invention a également pour objet l'utilisation à titre de principe actif dans une composition cosmétique ou pour la préparation d'une composition pharmaceutique d'une quantité efficace d'au moins un extrait tel que défini précédement pour combattre les désordres faisant intervenir un processus inflammatoire tel que décrit précédement.

De façon avantageuse, selon l'invention au moins un extrait d'au moins une Labiée du genre Rosmarinus peut être associé à des produits à effet irritant utilisés couramment dans le domaine cosmétique ou pharmaceutique, produits qui sont parfois des actifs cosmétiques ou pharmaceutiques. La présence d'un extrait d'au moins une Labiée du genre Rosmarinus dans une composition cosmétique ou pharmaceutique comprenant un produit ayant un effet irritant permet d'atténuer fortement, voire de supprimer cet effet irritant.
Cela permet en outre d'augmenter la quantité de principe actif à effet irritant par rapport à la quantité de principe actif normalement utilisée, en vue d'une efficacité améliorée.

L'invention concerne plus particulièrement une composition cosmétique ou pharmaceutique, caractérisée en ce qu'elle comprend, dans un milieu cosmétiquement ou pharmaceutiquement acceptable, au moins un produit à effet irritant et au moins un extrait d'au moins une Labiée du genre Rosmarinus.

Comme produits à effet irritant, on peut citer par exemple et sans limitation les tensioactifs (ioniques ou non-ioniques), les conservateurs, les solvants organiques ou les actifs comme les α-hydroxy-acides (acide citrique, malique, glycolique, tartrique, mandélique, lactique), les β-hydroxyacides (l'acide salicylique et ses dérivés), les α-céto-acides, les β-cétoacides, les rétinoïdes (rétinol, rétinal, acide rétinoïque), les anthralines (dioxyanthranol), les anthranoïdes, les peroxydes (notamment de benzoyle), le minoxidil, les sels de lithium, les antimétabolites, la vitamine D et ses dérivés, les teintures ou colorants capillaires (paraphénylènediamine et ses dérivés, les aminophénols), les solutions alcooliques parfumantes (parfums, eaux de toilette, après rasage, déodorants), les agents antitranspirants (certains sels d'aluminium), les actifs dépilatoires ou de permanentes (thiols), les actifs dépigmentants (hydroquinone).

Préférentiellement, l'invention concerne une composition cosmétique ou pharmaceutique, caractérisée en ce qu'elle comprend, dans un milieu cosmétiquement ou pharmaceutiquement acceptable, au moins un extrait d'au moins une Labiée du genre Rosmarinus et au moins un produit à effet irritant choisi parmi les actifs comme les α-hydroxy-acides (acide citrique, malique, glycolique, tartrique, mandélique, lactique), les β-hydroxyacides (l'acide salicylique et ses dérivés), les α-céto-acides, les β-cétoacides, les rétinoïdes (rétinol, rétinal, acide rétinoïque), les anthralines (dioxyanthranol), les anthranoïdes, les peroxydes (notamment de benzoyle), le minoxidil, les sels de lithium, les antimétabolites, la vitamine D et ses dérivés, les teintures ou colorants capillaires (paraphénylènediamine et ses dérivés, les aminophénols), les agents antitranspirants (certains sels d'aluminium), les actifs dépilatoires ou de permanentes (thiols), les actifs dépigmentants (hydroquinone).

L'emploi d'au moins un extrait d'au moins une Labiée du genre Rosmarinus permet notamment de multiplier de 2 à 10 fois la quantité de principe actif à effet irritant par rapport à l'état de la technique, sans ressentir tous les inconforts mentionnés ci-dessus. Ainsi, on peut utiliser les hydroxyacides jusqu'à 50 % du poids de la composition ou les rétinoïdes jusqu'à 5 %, en diminuant notablement leur caractère irritant.

La présente invention a en outre pour objet un procédé de traitement cosmétique en vue de diminuer l'effet irritant d'une composition cosmétique, caractérisé par le fait que l'on applique sur la peau, sur les cheveux, et/ou sur les muqueuses, une composition telle que décrite ci-dessus.

Le procédé de traitement cosmétique de l'invention peut être mis en oeuvre notamment en appliquant les compositions hygiéniques ou cosmétiques telles que définies ci-dessus, selon la technique d'utilisation habituelle de ces compositions. Par exemple : application de crèmes, de gels, de sérums, de lotions, de laits de démaquillage ou de compositions anti-solaires sur la peau ou sur les cheveux secs, application d'une lotion pour cheveux sur cheveux mouillés, de shampooings, ou encore application de dentifrice sur les gencives.

Les exemples et compositions suivants illustrent l'invention sans la limiter aucunement. Dans les compositions les proportions indiquées sont des pourcentages en poids.

### Exemple 1 : Préparation d'un extraits de cellules indifférenciées cultivées in vitro de Rosmarinus officinalis :

Sous azote, des cellules indifférenciées de *Rosmarinus officinalis* cultivées *in vitro* en conditions axéniques sont récupérées après culture en Erlenmeyer ou en fermenteur par filtration sur tamis de 50 µm. A 1 g de matière fraîche ainsi obtenue, on ajoute 1 ml d'eau déminéralisée contenant 1,8 g/l de cystéine. L'ensemble est broyé (Porter®, Ultra Turrax®...) au Turrax® à 24000 T/min durant 1 minute à 4°C (bain de glace). Le broyat est centrifugé 15 minutes à 10000 G à 4°C. Le surnageant est filtré à 0,22 µm (filtration stérilisante).
L'extrait ainsi préparé est conservé à 4°C. Il renferme environ 15 g de matière sèche par litre.

L'extrait est alors fractionné par ultrafiltration sur membrane de type Sartorius® afin d'en éliminer les phénomènes d'oxydation.

On obtient ainsi un extrait aqueux utilisable directement (extrait aqueux).

L'extrait est ensuite lyophilisé (extrait lyophilisé)

### Exemple 2 : Activités pharmacologiques des extraits de l'exemple 1 :

L'affinité réceptorielle des extraits de *Rosmarinus officinalis* a été testée pour les récepteurs B2, IL-1β, IL-6, TNF et H1 :

La mesure de l'affinité réceptorielle de l'extrait de *Rosmarinus officinalis* pour le récepteur B2 a été effectuée selon la méthode décrite dans l'article : Burch, R. M. et al., Biotech update (DuPont NEN), 7:3-11; (1992).

La mesure de l'affinité réceptorielle de l'extrait de *Rosmarinus offcinalis* pour le récepteur IL-1β a été effectuée selon la méthode décrite dans l'article : Bird, T. A. et al., FEBS Letter, 225 : 21-26 ; (1987).

La mesure de l'affinité réceptorielle de l'extrait de *Rosmarinus offcinalis* pour le récepteur IL-6 a été effectuée selon la méthode décrite dans l'article : Taga, T. et al., J. Exp. Med., 166 : 967 - 981 ; (1987).

La mesure de l'affinité réceptorielle de l'extrait de *Rosmarinus offcinalis* pour le récepteur TNF a été effectuée selon la méthode décrite dans l'article : Brockhaus, M. et al., P.N.A.S., 87 : 3127 - 3131 ; (1990).

La mesure de l'affinité réceptorielle de l'extrait de *Rosmarinus officinalis* pour le récepteur H1 a été effectuée selon la méthode décrite dans l'article : Dini S. et al., Agents and Actions, 33 : 181 - 184 ; (1991).

Les extraits préparés selon l'exemple 1 ont été testés aux concentrations de 0,5 %, 2 % et 5 %.

Lors de chaque expérience, la molécule de référence du récepteur étudié (NPC 567 pour le récepteur B2, IL-1β pour le récepteur IL-1β, IL-6 pour le récepteur IL-6, TNFα pour le récepteur TNF et la pyrilamine pour le récepteur H1) est parallèlement testée à 8 concentrations (n = 2) pour l'obtention d'une courbe standard permettant de valider l'expérimentation.

Les résultats des ces expériences sont résumés dans le tableau ci-après :
ces résultats sont exprimés en pourcentage d'inhibition de la fixation calculé par rapport au témoin.

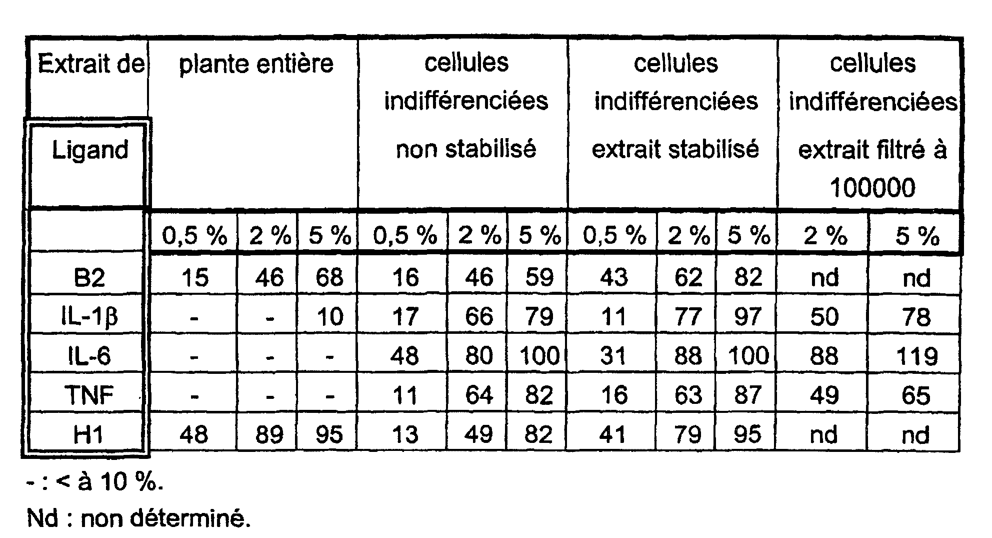

Les résultats de ces expériences mettent en évidence une affinité de l'extrait de *Rosmarinus officirialis* pour les récepteurs des médiateurs de l'inflammation.
Plus particulièrement les extraits de cellules indifférenciées présentent une bonne affinité pour plusieurs récepteurs des médiateurs de l'inflammation.
Les extraits stabilisés et/ou filtrés pour inhiber et/ou éliminer certaines enzymes, en particulier les oxydases, ont conservé leur affinité pour les récepteurs des médiateurs de l'inflammation.
Ces extraits sont de bons anti-inflammatoires pluripotents.

### Exemple 3 : Exemples de formulations illustrant l'invention et particulièrement les compositions selon l'invention associant au moins un extrait de Rosmarinus officinalis et un produit à effet irritant. Ces compositions ont été obtenues par simple mélange des différents composants.

### Composition 1 : Lotion démaquillante pour le visage

| | |
|---|---|
| Extrait de l'exemple 1 (forme aqueuse) | 10,00 % |
| Antioxydant | 0,05 % |
| Isopropanol | 40,00 % |
| Conservateur | 0,30 % |
| Eau | qsp 100,00 % |

### Composition 2 : Shampooing

| | |
|---|---|
| Extrait de l'exemple 1 (forme aqueuse) | 5,00 % |
| Hydroxypropylcellulose (Klucel H® vendu par la société Hercules) | 1,00 % |
| Parfum | 0,50 % |
| Conservateur | 0,30 % |
| Eau | qsp 100,00 % |

### Composition 3 : Crème de soin du visage (émulsion huile dans eau)

| | |
|---|---|
| Extrait de l'exemple 1 (forme lyophilisée) | 0,20 % |
| Stéarate de glycérol | 2,00 % |
| Polysorbate 60 (Tween 60® vendu par la société ICI) | 1,00 % |
| Acide stéarique | 1,40 % |
| Triéthanolamine | 0,70 % |
| Carbomer | 0,40 % |
| Fraction liquide du beurre de karité | 12,00 % |
| Perhydrosqualène | 12,00 % |
| Antioxydant | 0,05 % |
| Parfum | 0,50 % |
| Conservateur | 0,30 % |
| Eau | qsp 100,00 % |

### Composition 4 : Gel pour le traitement de l'acné

| | |
|---|---|
| Extrait de l'exemple 1 (forme lyophilisée) | 0,50 % |
| Acide tout trans rétinoïque | 0,05 % |
| Hydroxypropylcellulose (Klucel H® vendu par la société Hercules) | 1,00 % |
| Antioxydant | 0,05 % |
| Isopropanol | 40,00 % |
| Conservateur | 0,30 % |
| Eau | qsp 100,00 % |

### Composition 5 : Gel pour le soin du visage

| | |
|---|---|
| Extrait de l'exemple 1 (forme aqueuse) | 5,00 % |
| Hydroxypropylcellulose (Klucel H® vendu par la société Hercules) | 1,00 % |
| Antioxydant | 0,05 % |
| Isopropanol | 40,00 % |
| Conservateur | 0,30 % |
| Eau | qsp 100,00 % |

### Composition 6 : Gel anti-douleur

| | |
|---|---|
| Extrait de l'exemple 1 (forme aqueuse) | 10,00 % |
| Hydroxypropylcellulose (Klucel H® vendu par la société Hercules) | 1,00 % |
| Antioxydant | 0,05 % |
| Chlorhydrate de lidocaïne | 2,00 % |
| Isopropanol | 40,00 % |
| Conservateur | 0,30 % |
| Eau | qsp 100,00 % |

### Composition 7 : Crème de soin de l'érythème solaire (émulsion huile-dans-eau)

| | |
|---|---|
| Extrait de l'exemple 1 (forme lyophilisée) | 0,50 % |
| Stéarate de glycérol | 2,00 % |
| Polysorbate 60 (Tween 60® vendu par la société ICI) | 1,00 % |
| Acide stéarique | 1,40 % |
| Acide glycyrrhétinique | 2,00 % |
| Triéthanolamine | 0,70 % |
| Carbomer | 0,40 % |
| Fraction liquide du beurre de karité | 12,00 % |
| Huile de tournesol | 10,00 % |
| Antioxydant | 0,05 % |
| Parfum | 0,50 % |
| Conservateur | 0,30 % |
| Eau | qsp 100,00 % |

### Composition 8 : Crème de soin antirides pour le visage (émulsion huile/eau)

| | |
|---|---|
| Extrait de l'exemple 1 (forme aqueuse) | 5,00 % |
| Stéarate de glycérol | 2,00 % |
| Polysorbate 60 (Tween 60® vendu par la société ICI) | 1,00 % |
| Acide stéarique | 1,40 % |
| Acide n-octanoyl-5-salicylique | 0,50 % |
| Triéthanolamine | 0,70 % |
| Carbomer | 0,40 % |
| Fraction liquide du beurre de karité | 12,00 % |
| Perhydrosqualèrie | 12,00 % |
| Antioxydant | 0,05 % |
| Parfum | 0,50 % |
| Conservateur | 0,30 % |
| Eau | qsp 100,00 % |

### Composition 9 : Lotion pour éliminer les cicatrices dues à l'acné

| | |
|---|---|
| Extrait de l'exemple 1 (forme aqueuse) | 5,00 % |
| Acide glycolique | 50,00 % |
| Hydroxypropylcellulose (Klucel H® vendu par la société Hercules) | 0,05 % |
| Conservateur | 0,30 % |
| NaOH | qsp pH = 2,8 |
| Ethanol | qsp 100,00 % |

## Revendications

1. Utilisation dans une composition cosmétique ou pour la préparation d'une composition pharmaceutique, à titre de principe actif pour combattre les désordres faisant intervenir un processus inflammatoire d'une quantité efficace d'au moins un extrait d'au moins un végétal de la famille des Labiées du genre Rosmarinus cultivé *in vitro.*

2. Utilisation selon la revendication précédente, **caractérisé en ce que** le végétal est du *Rosmarinus officinalis.*

3. Utilisation selon l'une quelconque des revendications précédentes, **caractérisé en ce que** c'est un extrait de cellules végétales.

4. Utilisation selon l'une quelconque des revendications précédentes, **caractérisé en ce que** les cellules végétales sont des cellules indifférenciées.

5. Utilisation selon l'une quelconque des revendications précédentes, **caractérisé en ce que** l'extrait est fractionné pour en éliminer les oxydases.

6. Utilisation selon l'une quelconque des revendications précédentes, **caractérisé en ce que** l'extrait est stabilisé.

7. Utilisation dans une composition cosmétique selon l'une quelconque des revendications précédentes, **caractérisée par le fait que** ledit extrait est en une quantité représentant de 0,001 % à 50 % du poids total de la composition et préférentiellement en une quantité représentant de 0,005 % à 25 % du poids total de la composition.

8. Utilisation dans une composition pharmaceutique selon l'une quelconque des revendications 1 à 9, **caractérisée par le fait que** ledit extrait est en une quantité représentant de 0,01 % à 70 % du poids total de la composition et préférentiellement en une quantité représentant de 0,05 % à 40 % du poids total de la composition.

9. Utilisation selon l'une quelconque des revendications précédentes pour traiter les désordres inflammatoires cutanés.

10. Utilisation selon l'une quelconque des revendications précédentes pour combattre les affections rhumatismales, les affections pulmonaires, les affections articulaires, les phases inflammatoires de l'alopécie, les affections cutanées, les piqûres d'insectes.

11. Utilisation selon l'une quelconque des revendications précédentes, pour combattre les irritations cutanées et/ou les dartres et/ou les sensations dysesthésiques et/ou les sensations d'échauffement et/ou les prurits de la peau et/ou des muqueuses.

12. Utilisation selon l'une quelconque des revendications précédentes, **caractérisée en ce que** la composition comprend en outre, dans un milieu cosmétiquement ou pharmaceutiquement acceptable, au moins un produit à effet irritant.

13. Composition cosmétique ou pharmaceutique, **caractérisée en ce qu'**elle comprend, dans un milieu cosmétiquement ou pharmaceutiquement acceptable, au moins un extrait d'au moins un végétal de la famille des Labiées du genre Rosmarinus tel que décrit dans l'une quelconque des revendications 1 à 8 et au moins un produit à effet irritant, le dit un extrait d'au moins un végétal de la famille des Labiées du genre Rosmarinus est présent dans la composition en une quantité suffisante pour éliminer l'effet irritant dudit produit à effet irritant.

14. Composition selon la revendication 13, **caractérisée en ce que** le produit à effet irritant est choisi parmi les tensioactifs (ioniques ou non-ioniques), les conservateurs, les solvants organiques ou les actifs comme les α-hydroxy-acides (acide citrique, malique, glycolique, tartrique, mandélique, lactique), les β-hydroxyacides (l'acide salicylique et ses dérivés), les α-céto-acides, les β-cétoacides, les rétinoïdes (rétinol, rétinal, acide rétinoïque), les anthralines (dioxyanthranol), les anthranoïdes, les peroxydes (notamment de benzoyle), le minoxidil, les sels de lithium, les antimétabolites, la vitamine D et ses dérivés, les teintures ou colorants capillaires (paraphénylénediamine et ses dérivés, les aminophénols), les solutions alcooliques parfumantes (parfums, eaux de toilette, après rasage, déodorants), les agents antitranspirants (certains sels d'aluminium), les actifs dépilatoires ou de permanentes (thiols), les actifs dépigmentants (hydroquinone).

15. Composition selon la revendication 14 **caractérisée en ce que** le produit à effet irritant est choisi parmi les actifs comme les α-hydroxy-acides (acide citrique, malique, glycolique, tartrique, mandélique, lactique), les β-hydroxyacides (l'acide salicylique et ses dérivés), les α-céto-acides, les β-cétoacides, les rétinoïdes (rétinol, rétinal, acide rétinoïque), les anthranoïdes, les peroxydes (notamment de benzoyle), le minoxidil, les sels de lithium, les antimétabolites, la vitamine D et ses dérivés, les teintures ou colorants capillaires (paraphénylènediamine et ses dérivés, les aminophénols), les agents antitranspirants (certains sels d'aluminium), les actifs dépilatoires ou de permanentes (thiols), les actifs dépigmentants (hydroquinone).

## Claims

1. Use, in a cosmetic composition or for the preparation of a pharmaceutical composition, as active ingredient for controlling disorders involving an inflammatory process, of an effective quantity of at least one extract of at least one plant of the Labiatae family, of the genus Rosmarinus cultured *in vitro.*

2. Use according to the preceding claim, **characterized in that** the plant is *Rosmarinus officinalis.*

3. Use according to any one of the preceding claims, **characterized in that** it is an extract of plant cells.

4. Use according to any one of the preceding claims, **characterized in that** the plant cells are undifferentiated cells.

5. Use according to any one of the preceding claims, **characterized in that** the extract is fractionated so as to remove oxidases therefrom.

6. Use according to any one of the preceding claims, **characterized in that** the extract is stabilized.

7. Use in a cosmetic composition according to any one of the preceding claims, **characterized in that** the said extract is in a quantity representing from 0.001% to 50% of the total weight of the composition and preferably in a quantity representing from 0.005% to 25% of the total weight of the composition.

8. Use in a pharmaceutical composition according to any one of Claims 1 to 6, **characterized in that** the said extract is in a quantity representing from 0.01% to 70% of the total weight of the composition and preferably in a quantity representing from 0.05% to 40% of the total weight of the composition.

9. Use according to any one of the preceding claims, for treating cutaneous inflammatory disorders.

10. Use according to any one of the preceding claims, for controlling rheumatic conditions, pulmonary conditions, articular conditions, the inflammatory phases of alopecia, skin conditions, insect bites.

11. Use according to any one of the preceding claims, for controlling skin irritations and/or dartres and/or dysesthetic sensations and/or sensations of overheating and/or pruritus of the skin and/or of the mucous membranes.

12. Use according to any one of the preceding claims, **characterized in that** the composition comprises in addition, in a cosmetically or pharmaceutically acceptable medium, at least one product with an irritant effect.

13. Cosmetic or pharmaceutical composition, **characterized in that** it comprises, in a cosmetically or pharmaceutically acceptable medium, at least one extract of at least one plant of the Labiatae family, of the genus Rosmarinus, as described in any one of Claims 1 to 8 and at least one product with an irritant effect, the said one extract of at least one plant of the Labiatae family, of the genus Rosmarinus, is present in the composition in a sufficient quantity to eliminate the irritant effect of the said product with an irritant effect.

14. Composition according to Claim 13, **characterized in that** the product with an irritant effect is chosen from surfactants (ionic or nonionic), preservatives, organic solvents or active agents such as α-hydroxy acids (citric, malic, glycolic, tartaric, mandelic or lactic acid), β-hydroxy acids (salicylic acid and its derivatives), α-keto acids, β-keto acids, retinoids (retinol, retinal, retinoic acid), anthralins (dioxyanthranol), anthranoids, peroxides (particularly benzoyl peroxide), minoxidil, lithium salts, antimetabolites, vitamin D and its derivatives, hair dyes or colorants (para-phenylenediamine and its derivatives, aminophenols), perfuming alcoholic solutions (perfume, toilet water, aftershave, deodorants), anti-perspirants (some aluminium salts), depilatory active agents or permanent-waving active agents (thiols), depigmenting active agents (hydroquinone).

15. Composition according to Claim 14, **characterized in that** the product with an irritant effect is chosen from active agents such as α-hydroxy acids (citric, malic, glycolic, tartaric, mandelic or lactic acid), β-hydroxy acids (salicylic acid and its derivatives), α-keto acids, β-keto acids, retinoids (retinol, retinal, retinoic acid), anthranoids, peroxides (particularly benzoyl peroxide), minoxidil, lithium salts, antimetabolites, vitamin D and its derivatives, hair dyes or colorants (para-phenylenediamine and its derivatives, aminophenols), anti-perspirants (some aluminium salts), depilatory active agents or permanent-waving active agents (thiols), depigmenting active agents (hydroquinone).

## Patentansprüche

1. Verwendung einer wirksamen Menge mindestens eines Extraktes mindestens einer *in-vitro* kultivierten Pflanze der Gattung Rosmarinus aus der Familie der Labiatae als Hauptwirkstoff in einer kosmetischen Zusammensetzung oder zur Herstellung einer pharmazeutischen Zusammensetzung, um Störungen zu bekämpfen, an denen entzündliche Prozesse beteiligt sind.

2. Verwendung nach dem vorhergehenden Anspruch, **dadurch gekennzeichnet, daß** es sich bei der Pflanze um *Rosmarinus officinalis* handelt.

3. Verwendung nach einem der vorhergehenden Ansprüche, **dadurch gekennzeichnet, daß** es sich um einen Extrakt von Pflanzenzellen handelt.

4. Verwendung nach einem der vorhergehenden Ansprüche, **dadurch gekennzeichnet, daß** die Pflanzenzellen undifferenzierte Zellen sind.

5. Verwendung nach einem der vorhergehenden Ansprüche, **dadurch gekennzeichnet, daß** der Extrakt fraktioniert wurde, um die Oxydasen zu entfernen.

6. Verwendung nach einem der vorhergehenden Ansprüche, **dadurch gekennzeichnet, daß** der Extrakt stabilisiert ist.

7. Verwendung nach einem der vorhergehenden Ansprüche in einer kosmetischen Zusammensetzung, **dadurch gekennzeichnet, daß** der Extrakt in einer Menge vorliegt, die 0,001 bis 50 % des Gesamtgewichts der Zusammensetzung und vorzugsweise 0,005 bis 25 % des Gesamtgewichts der Zusammensetzung ausmacht.

8. Verwendung nach einem der Ansprüche 1 bis 6 in einer pharmazeutischen Zusammensetzung, **dadurch gekennzeichnet, daß** der Extrakt in einer Menge vorliegt, die 0,01 bis 70 % des Gesamtgewichts der Zusammensetzung und vorzugsweise 0,05 bis 40 % des Gesamtgewichts der Zusammensetzung ausmacht.

9. Verwendung nach einem der vorhergehenden Ansprüche zur Behandlung von entzündlichen Störungen der Haut.

10. Verwendung nach einem der vorhergehenden Ansprüche zur Behandlung von rheumatischen Erkrankungen, pulmonalen Erkrankungen, artikulären Erkrankungen, entzündlichen Phasen der Alopezie, Hautleiden und Insektenstichen.

11. Verwendung nach einem der vorhergehenden Ansprüche zur Behandlung von Hautirritationen und/oder Flechten und/oder dysästhesischen Empfindungen und/oder Hitzegefühlen und/oder Juckreiz der Haut und/oder der Schleimhäute.

12. Verwendung nach einem der vorhergehenden Ansprüche, **dadurch gekennzeichnet, daß** die Zusammensetzung in einem kosmetisch oder pharmazeutisch akzeptablen Medium ferner ein Produkt mit reizender Nebenwirkung enthält.

13. Kosmetische oder pharmazeutische Zusammensetzung, **dadurch gekennzeichnet, daß** sie in einem kosmetisch oder pharmazeutisch akzeptablen Medium mindestens einen Extrakt mindestens einer. Pflanze der Gattung Rosmarinus aus der Familie der Labiatae nach einem der Ansprüche 1 bis 8 und mindestens ein Produkt mit reizender Nebenwirkung enthält, wobei der Extrakt mindestens einer Pflanze der Gattung Rosmarinus aus der Familie der Labiatae in der Zusammensetzung in einer Menge vorliegt, die ausreichend ist, um die reizende Wirkung des Produktes mit reizender Wirkung zu beseitigen.

14. Zusammensetzung nach Anspruch 13, **dadurch gekennzeichnet, daß** das Produkt mit reizender Wirkung unter den grenzflächenaktiven Stoffen (ionischen oder nichtionischen grenzflächenaktiven Stoffen), Konservierungsmitteln, organischen Lösungsmitteln oder Wirkstoffen, wie α-Hydroxysäuren (Citronensäure, Äpfelsäure, Glykolsäure, Weinsäure, Mandelsäure, Milchsäure), β-Hydroxysäuren (Salicylsäure und ihren Derivaten), α-Ketosäuren, β-Ketosäuren, Retinoiden (Retinol, Retinal, Retinsäure), Anthralinen (Dioxyanthranol), Anthranoiden, Peroxiden (insbesondere Benzoylperoxid), Minoxidil, Lithiumsalzen, Antimetaboliten, Vitamin D und seinen Derivaten, Haarfärbemitteln oder Farbstoffen für das Haar (p-Phenylendiamin und seinen Derivaten, Aminophenolen), parfümierenden alkoholischen Lösungen (Parfums, Eau de Toilette, After Shave, Deodorants), Antitranspirantien (verschiedenen Aluminiumsalzen), Wirkstoffen zur Haarentfernung oder für Dauerwellen (Thiolen) und depigmentierenden Wirkstoffen (Hydrochinon), ausgewählt ist.

15. Zusammensetzung nach Anspruch 14, **dadurch gekennzeichnet, daß** das Produkt mit reizender Wirkung unter den Wirkstoffen, wie α-Hydroxysäuren (Citronensäure, Äpfelsäure, Glykolsäure, Weinsäure, Mandelsäure, Milchsäure), β-Hydroxysäuren (Salicylsäure und ihren Derivaten), α-Ketosäuren, β-Ketosäuren, Retinoiden (Retinol, Retinal, Retinsäure), Anthranoiden, Peroxiden (insbesondere Benzoylperoxid), Minoxidil, Lithiumsalzen, Antimetaboliten, Vitamin D und seinen Derivaten, Haarfärbemitteln oder Farbstoffen für das Haar (p-Phenylendiamin und seinen Derivaten, Aminophenolen), Antitranspirantien (verschiedenen Aluminiumsalzen), Wirkstoffen zur Haarentfernung oder für Dauerwellen (Thiolen) und depigmentierenden Wirkstoffen (Hydrochinon), ausgewählt ist.
